(19) Europäisches Patentamt
European Patent Office
Office européen des brevets

(11) Veröffentlichungsnummer: **0 427 059 A2**

# (12) EUROPÄISCHE PATENTANMELDUNG

(21) Anmeldenummer: **90120546.8**

(22) Anmeldetag: **26.10.90**

(51) Int. Cl.5: **C07D 405/06**, A01N 43/50, A01N 43/653

(30) Priorität: **04.11.89 DE 3936824**

(43) Veröffentlichungstag der Anmeldung: **15.05.91 Patentblatt 91/20**

(84) Benannte Vertragsstaaten: **AT BE CH DE DK ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Seele, Rainer, Dr.**
**Leiblstrasse 3**
**W-6701 Fussgoenheim(DE)**
Erfinder: **Hickmann, Eckhardm Dr.**
**Kantstrasse 23**
**W-6701 Dannstadt-Schauernheim(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**

## (54) N-Oxo-Azolylmethyloxirane und diese enthaltende Fungizide und Bioregulatoren.

(57) N-Oxo-Azolylmethyloxirane der Formel I

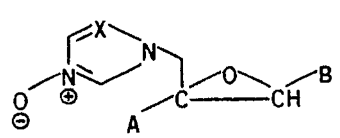

I,

in welcher A und B Alkyl, Cycloalkyl, Tetrahydropyranyl, Benzyl, Norbornyl, Naphthyl, Biphenyl oder Phenyl bedeuten, wobei diese Reste substituiert sein können und X für CH oder N steht sowie diese enthaltende Fungizide und Bioregulatoren.

EP 0 427 059 A2

## N-OXO-AZOLYLMETHYLOXIRANE UND DIESE ENTHALTENDE FUNGIZIDE UND BIOREGULATOREN

Die vorliegende Erfindung betrifft neue N-Oxo-Azolylmethyloxirane der allgemeinen Formel I

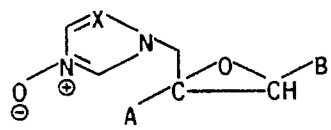

I,

in welcher

A und B gleich oder verschieden $C_1$-$C_8$-Alkyl, $C_3$-$C_8$-Cycloalkyl, Tetrahydropyranyl, Norbornyl, Naphthyl, Biphenyl oder Phenyl bedeuten, wobei diese Reste unsubstituiert oder ein- bis dreifach durch Halogen, Nitro, Phenoxy, $C_1$-$C_4$-Alkyl, $C_1$-$C_4$-Alkoxy oder $C_1$-$C_4$-Halogenalkyl substituiert sind und

X für CH oder N steht.

Weiterhin betrifft die Erfindung ein Verfahren zur Herstellung der Verbindungen I und diese enthaltende Fungizide.

Aus den europäischen Offenlegungsschriften EP-A 94 564, 196 038 und 315 850 (DE-A 37 37 888) ist bekannt, daß Azolylmethyloxirane als Pflanzenschutzmittel, insbesondere Fungizide und Bioregulatoren verwendet werden.

Der Erfindung lag nun die Aufgabe zugrunde, neue Verbindungen mit einem ähnlichen Wirkungsspektrum und möglichst verbesserter biologischer Wirkung bereitzustellen.

Demgemäß werden die eingangs definierten N-Oxo-azolylmethyloxirane gefunden, die eine bessere fungizide Wirkung besitzen als bekannte Azolverbindungen und sich zur Regulierung des Pflanzenwachstums eignen.

Im einzelnen haben die Substituenten in Formel I folgende Bedeutung:

A und B unabhängig voneinander

- unverzweigtes oder verzweigtes $C_1$-$C_8$-Alkyl, insbesondere $C_1$-$C_4$-Alkyl wie Methyl, Ethyl, Propyl, Isopropyl, Butyl, iso-Butyl, sec.-Butyl, tert.-Butyl,
- $C_3$-$C_8$-Cycloalkyl wie Cyclopropyl, Cyclopentyl, Cyclohexyl,
- Tetrahydropyranyl wie 2-Tetrahydropyranyl, 3-Tetrahydropyranyl, 4-Tetrahydropyranyl,
- Benzyl, Phenyl,
- Norbornyl,
- Naphthyl wie 1-Naphthyl oder 2-Naphthyl,
- Biphenyl wie o-, m- oder p-Biphenyl.

Die genannten Reste können ein- bis dreifach substituiert sein durch

- Halogen wie Fluor, Chlor oder Brom,
- Nitro,
- Phenoxy,
- $C_1$-$C_4$-Alkyl wie oben im einzelnen genannt,
- $C_1$-$C_4$-Alkoxy, z.B. Methoxy oder Ethoxy,
- $C_1$-$C_4$-Halogenalkyl mit 1 bis 3 Halogenatomen wie Fluor, Chlor oder Brom.

Vorzugsweise stehen A und B für Phenyl oder durch ein, zwei oder drei Halogenatome substituiertes Phenyl, wie 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2,3-Difluorphenyl, 2,4-Difluorphenyl, 2,5-Difluorphenyl, 2,6-Difluorphenyl, 3,4-Difluorphenyl, 3,5-Difluorphenyl, 2,3-Difluorphenyl, 2,4-Dichlorphenyl, 2,5-Dichlorphenyl, 2,6-Dichlorphenyl, 3,4-Dichlorphenyl, 3,5-Dichlorphenyl, 2,3-Dibromphenyl, 2,4-Dibromphenyl, 2,5-Dibromphenyl, 2,6-Dibromphenyl, 3,4-Dibromphenyl, 3,5-Dibromphenyl, 2-Chlor-3-fluorphenyl, 3-Chlor-2-fluorphenyl, 2-Chlor-4-fluorphenyl, 4-Chlor-2-fluorphenyl, 2-Chlor-5-fluorphenyl, 5-Chlor-2-fluorphenyl, 2-Chlor-6-fluorphenyl, 3-Chlor-4-fluorphenyl, 4-Chlor-3-fluorphenyl, 3-Chlor-5-fluorphenyl, 2-Brom-3-fluorphenyl, 3-Brom-2-fluorphenyl, 2-Brom-4-fluorphenyl, 4-Brom-2-fluorphenyl, 2-Brom-5-fluorphenyl, 5-Brom-2-fluorphenyl, 2-Brom-6-fluorphenyl, 3-Brom-4-fluorphenyl, 4-Brom-3-fluorphenyl, 3-Brom-5-fluorphenyl, 2-Brom-3-chlorphenyl, 3-Brom-2-chlorphenyl, 2-Brom-4-chlorphenyl, 4-Brom-2-chlorphenyl, 2-Brom-5-fluorphenyl, 5-Brom-2-chlorphenyl, 4-Brom-6-chlorphenyl, 3-Brom-4-chlorphenyl, 4-Brom-3-chlorphenyl, 3-Brom-5-chlorphenyl.

Folgende Reste sind besonders bevorzugt: Methyl, Ethyl, Isopropyl, n-Propyl, n-Butyl, sec.-Butyl, tert.-Butyl, n-Pentyl, Neopentyl, Phenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl,

2,5-Dichlorphenyl, 2,6-Dichlorphenyl, 2-Chlor-6-fluorphenyl, 2-Methoxyphenyl, 3-Methoxyphenyl, 4-Methoxyphenyl, 2,4-Dimethoxyphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenyoxyphenyl, 3-Nitrophenyl, 4-Nitrophenyl, 2-Trifluormethylphenyl, 3-Trifluoromethylphenyl, 4-Trifluoromethylphenyl.

Die Verbindungen der Formel I enthalten chirale Zentren und werden im allgemeinen in Form von Racematen bzw. als Diastereomerengemische von erythro- bzw. threo-Formen erhalten. Die erythro- bzw. threo-Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Aus einem solchen isolierten Diastereomeren kann man mit bekannten Methoden einheitliche Enantiomere erhalten. Als fungizide Mittel kann man sowohl die einheitlichen Diastereomere bzw. Enantiomere wie auch deren bei der Synthese anfallende Gemische verwenden.

Die N-Oxo-azolylmethyloxirane der Formel I können z.B. hergestellt werden, indem man entsprechend substituierte Azolylmethyloxirane der Formel II

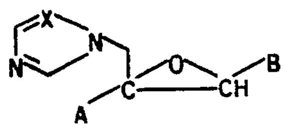

II,

in der A, B und X die für das N-Oxid I angegebene Bedeutung haben, mit Peroxycarbonsäuren, $H_2O_2$ oder Alkylhydroperoxiden umsetzt und in an sich bekannter Weise isoliert.

Geeignete Peroxycarbonsäuren sind z.B. Perbenzoesäure, 3-Chlorperbenzoesäure, 4-Nitroperbenzoesäure, Monoperphthalsäure, Peressigsäure, Perpropionsäure, Monopermaleinsäure, Monoperbernsteinsäure, Perpelargonsäure oder Trifluorperessigsäure.

Die Peroxycarbonsäuren können auch in situ aus dem entsprechenden Säureanhydrid und Wasserstoffperoxid hergestellt werden. Beispielsweise läßt sich Permaleinsäure aus Maleinsäureanhydrid und Wasserstoffperoxid, z.B. einer 30- bis 50-gew.-%igen wäßrigen Wasserstoffperoxidlösung, herstellen. Im allgemeinen liegen die Molverhältnisse Anhydrid zu $H_2O_2$ bei etwa 1,5 zu 10, insbesondere 2 bis 4.

Die Oxidation mit Persäuren erfolgt in aprotisch-polaren Lösungsmitteln, vorzugsweise chlorierten Kohlenwasserstoffen, z.B. Methylenchlorid, Chloroform, Tetrachlorkohlenstoff, Dichlorethan, aber gegebenenfalls auch in Essigsäure, Essigester, Aceton oder Dimethylformamid. In manchen Fällen kann es vorteilhaft sein, dem Reaktionsgemisch einen üblichen Puffer wie Natriumacetat, Natriumcarbonat, Dinatriumhydrogenphosphat oder Benzyl-trimethylammoniumhydroxid (Triton® B) hinzuzufügen.

Die Reaktionstemperatur liegt bei 10 bis 100, insbesondere 20 bis 80 °C.

Die Oxidation kann in Abwesenheit oder in Gegenwart eines Katalysators wie Iod, Pyridin-N-oxid, Natriumwolframat oder Licht vorgenommen werden.

Als Oxidationsmittel eignen sich auch alkalische Lösungen von Wasserstoffperoxid, z.B. 30- bis 50-gew.-%ige wäßrige Lösungen. In diesem Fall kommen als Lösungsmittel Alkohole wie Methanol oder Ethanol sowie Aceton oder Acetonitril in Frage. Die Reaktionstemperatur liegt in der Regel niedriger als im Fall von Persäuren als Oxidationsmittel und beträgt etwa 10 bis 50, z.B. 25 bis 30 °C.

Weiterhin können Alkylhydroperoxide, z.B. tert.-Butylhydroperoxid oder Cyclohexylhydroperoxid als Oxidationsmittel verwendet werden. In diesem Fall empfiehlt sich der Zusatz eines Katalysators wie z.B. Natriumwolframat, Perwolframsäure, Molybdänhexacarbonyl oder Vanadylacetylacetonat. Als Lösungsmittel können die obengenannten aprotisch-polaren Lösungsmittel verwendet werden wie z.B. chlorierte Kohlenwasserstoffe.

Bevorzugte Oxidationsmittel sind Peroxicarbonsäuren, insbesondere Permaleinsäure und m-Chlorperbenzoesäure.

Die Oxidationsmittel können in Mengen von etwa 1 bis 10, insbesondere 2 bis 8 mol im Fall von Peroxicarbonsäuren, von etwa 1 bis 20 mol im Fall von Wasserstoffperoxid und etwa 1 bis 20 mol im Fall von Alkylhydroperoxiden verwendet werden, wobei die Angaben jeweils bezogen sind auf 1 mol Ausgangsstoff II.

Die Isolierung der N-Oxide I aus dem Reaktionsgemisch erfolgt in üblicher Weise, z.B. indem man das Reaktionsgemisch einer chromatographischen Reinigung an Kieselgel unterwirft.

Der Ausgangsstoff II ist nach bekannten Verfahren, z.B. wie in EP-A-94 564, EP-A-196 038 oder EP-A-315 850 beschrieben, zugänglich.

Das folgende Beispiel erläutert die Herstellung der Wirkstoffe.

2-(1,2,4-Triazol-4-oxo-1-ylmethyl)-2-(4-fluorphenyl)-3-(2-chlor-phenyl)-oxiran

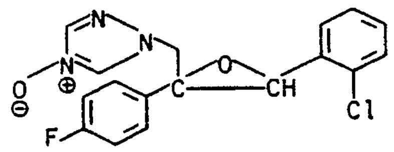

Zu einer Lösung von 20 g (0,06 mol) 2-(1,2,4-Triazol-1-yl-methyl)-2-(4-fluorphenyl)-3-(2-chlorphenyl)-oxiran und 0,5 ml Pyridin-N-Oxid in 150 ml 1,2-Dichlorethan werden bei Raumtemperatur 59,4 g (0,60 mol) Maleinsäureanhydrid gegeben. Das Reaktionsgemisch wird auf 0° C gekühlt und langsam mit 16,5 g (0,24 mol) Wasserstoffperoxid (ca. 50 %ig) versetzt. Nach beendeter Zugabe wird zwei Stunden bei 40-50° C gerührt und die Bildung des N-Oxyds durch Dünnschichtchromatographie verfolgt. Anschließend wird die ausgefallene Maleinsäure abgesaugt und das Filtrat mit Natriumhydrogencarbonat-Lösung und Wasser gewaschen, über Natriumsulfat getrocknet und im Vakuum eingedampft. Die folgende chromatographische Reinigung des verbleibenden Rückstandes an Kieselgel (n-Hexan/Essigester = 4:1 zur Isolierung des Eduktes; Essigester/Methanol = 1:1 zur Isolierung des N-Oxyds) liefert 6,3 g (30 % der Theorie) 2-(1,2,4-Triazol-4-oxo-1-ylmethyl)-2-(4-fluorphenyl)-3-(2-chlor-phenyl)-oxiran mit dem Schmelzpunkt 216-218° C.

Entsprechend Beispiel 1 können die in der Tabelle aufgeführten Verbindungen hergestellt werden.

Tabelle: N-Oxo-Azolylmethyloxirane I

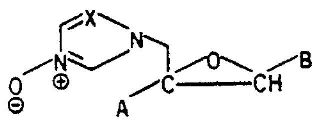

(I)

| Bsp | A | B | X | physikalische Daten Schmp./IR |
|---|---|---|---|---|
| 1 | 4-Fluorphenyl | 2-Chlorphenyl | N | 216-218°C |
| 2 | 4-Fluorphenyl | 2-Chlorphenyl | CH | |
| 3 | 4-Fluorphenyl | 3-Chlorphenyl | N | |
| 4 | 4-Fluorphenyl | 3-Chlorphenyl | CH | |
| 5 | 4-Fluorphenyl | 4-Chlorphenyl | N | |
| 6 | 4-Fluorphenyl | 4-Chlorphenyl | CH | |
| 7 | 4-Fluorphenyl | Phenyl | CH | |
| 8 | 4-Fluorphenyl | Phenyl | N | |
| 9 | 4-Fluorphenyl | 2,4-Dichlorphenyl | N | |
| 10 | 4-Fluorphenyl | 2,4-Dichlorphenyl | CH | |
| 11 | 4-Fluorphenyl | 2-Chlor-4-fluorphenyl | N | |
| 12 | 4-Fluorphenyl | 2-Chlor-4-fluorphenyl | CH | |
| 13 | 4-Fluorphenyl | 2-Fluorphenyl | N | |
| 14 | 4-Fluorphenyl | 2-Fluorphenyl | CH | |
| 15 | 4-Fluorphenyl | 3-Fluorphenyl | N | |
| 16 | 4-Fluorphenyl | 3-Fluorphenyl | CH | |
| 17 | 4-Fluorphenyl | 4-Fluorphenyl | N | |
| 18 | 4-Fluorphenyl | 4-Fluorphenyl | CH | |
| 19 | 4-Fluorphenyl | 2,4-Difluorphenyl | N | |
| 20 | 4-Fluorphenyl | 2,4-Difluorphenyl | CH | |
| 21 | 4-Fluorphenyl | 2-Bromphenyl | N. | |
| 22 | 4-Fluorphenyl | 2-Bromphenyl | CH | |
| 23 | 4-Fluorphenyl | 3-Bromphenyl | N | |
| 24 | 4-Fluorphenyl | 3-Bromphenyl | CH | |
| 25 | 4-Fluorphenyl | 4-Bromphenyl | N | |
| 26 | 4-Fluorphenyl | 4-Bromphenyl | CH | |
| 27 | 4-Fluorphenyl | 2-Methylphenyl | N | |
| 28 | 4-Fluorphenyl | 2-Methylphenyl | CH | |
| 29 | 4-Fluorphenyl | 4-Methylphenyl | N | |
| 30 | 4-Fluorphenyl | 4-Methylphenyl | CH | |
| 31 | 4-Fluorphenyl | 2,4-Dimethylphenyl | N | |
| 32 | 4-Fluorphenyl | 2,4-Dimethylphenyl | CH | |
| 33 | 4-Fluorphenyl | 4-tert.-Butylphenyl | N | |
| 34 | 4-Fluorphenyl | 2-Methoxyphenyl | N | |

| Bsp | A | B | X | physikalische Daten Schmp./IR |
|---|---|---|---|---|
| 35 | 4-Fluorphenyl | 2-Methoxyphenyl | CH | |
| 36 | 4-Fluorphenyl | 4-Methoxyphenyl | N | |
| 37 | 4-Fluorphenyl | 4-Methoxyphenyl | CH | |
| 38 | 4-Fluorphenyl | 4-Phenoxyphenyl | N | |
| 39 | 4-Fluorphenyl | 4-Phenoxyphenyl | CH | |
| 40 | 4-Fluorphenyl | 4-Nitrophenyl | N | |
| 41 | 4-Fluorphenyl | 4-Nitrophenyl | CH | |
| 42 | 4-Fluorphenyl | 2-Trifluormethylphenyl | N | |
| 43 | 4-Fluorphenyl | 2-Trifluormethylphenyl | CH | |
| 44 | 4-Fluorphenyl | 3-Trifluormethylphenyl | N | |
| 45 | 4-Fluorphenyl | 3-Trifluormethylphenyl | CH | |
| 46 | 4-Fluorphenyl | 4-Trifluormethylphenyl | N | |
| 47 | 4-Fluorphenyl | 4-Trifluormethylphenyl | CH | |
| 48 | 4-Fluorphenyl | 1-Naphthyl | N | |
| 49 | 4-Fluorphenyl | 1-Naphthyl | CH | |
| 50 | 4-Fluorphenyl | 2-Naphthyl | N | |
| 51 | 4-Fluorphenyl | 2-Naphthyl | CH | |
| 52 | 4-Fluorphenyl | 4-Biphenyl | N | |
| 53 | 4-Fluorphenyl | 4-Biphenyl | CH | |
| 54 | 4-Fluorphenyl | 4-Tetrahydropyranyl | N | |
| 55 | 4-Fluorphenyl | 4-Tetrahydropyranyl | CH | |
| 56 | 4-Fluorphenyl | Cyclopropyl | N | |
| 57 | 4-Fluorphenyl | Cyclopropyl | CH | |
| 58 | 4-Fluorphenyl | Cyclopentyl | N | |
| 59 | 4-Fluorphenyl | Cyclopentyl | CH | |
| 60 | 4-Fluorphenyl | Cyclohexyl | N | |
| 61 | 4-Fluorphenyl | Cyclohexyl | CH | |
| 62 | 4-Fluorphenyl | Norbornyl | N | |
| 63 | 4-Fluorphenyl | Norbornyl | CH | |
| 64 | Phenyl | Phenyl | N | |
| 65 | Phenyl | Phenyl | CH | |
| 66 | Phenyl | 2-Chlorphenyl | N | 194-196°C |
| 67 | Phenyl | 2-Chlorphenyl | CH | |
| 68 | Phenyl | 3-Chlorphenyl | N | |
| 69 | Phenyl | 3-Chlorphenyl | CH | |
| 70 | Phenyl | 4-Chlorphenyl | N | |
| 71 | Phenyl | 4-Chlorphenyl | CH | |
| 72 | Phenyl | 2,4-Dichlorphenyl | N | |
| 73 | Phenyl | 2,4-Dichlorphenyl | CH | |
| 74 | Phenyl | 2-Chlor-4-fluorphenyl | N | |
| 75 | Phenyl | 2-Chlor-4-fluorphenyl | CH | |

| Bsp | A | B | X | physikalische Daten Schmp./IR |
|---|---|---|---|---|
| 76 | Phenyl | 2-Fluorphenyl | N | |
| 77 | Phenyl | 2-Fluorphenyl | CH | |
| 78 | Phenyl | 3-Fluorphenyl | N | |
| 79 | Phenyl | 3-Fluorphenyl | CH | |
| 80 | Phenyl | 4-Fluorphenyl | N | |
| 81 | Phenyl | 4-Fluorphenyl | CH | |
| 82 | Phenyl | 2-Bromphenyl | N | |
| 83 | Phenyl | 2-Bromphenyl | CH | |
| 84 | Phenyl | 3-Bromphenyl | N | |
| 85 | Phenyl | 3-Bromphenyl | CH | |
| 86 | Phenyl | 4-Bromphenyl | N | |
| 87 | Phenyl | 4-Bromphenyl | CH | |
| 88 | Phenyl | 2-Methylphenyl | N | |
| 89 | Phenyl | 2-Methylphenyl | CH | |
| 90 | Phenyl | 4-Methylphenyl | N | |
| 91 | Phenyl | 4-Methylphenyl | CH | |
| 92 | Phenyl | 2,4-Dimethylphenyl | N | |
| 93 | Phenyl | 2,4-Dimethylphenyl | CH | |
| 94 | Phenyl | 2-Methoxyphenyl | N | |
| 95 | Phenyl | 2-Methoxyphenyl | CH | |
| 96 | Phenyl | 4-Phenoxyphenyl | N | |
| 97 | Phenyl | 4-Phenoxyphenyl | CH | |
| 98 | Phenyl | 2-Trifluormethylphenyl | N | |
| 99 | Phenyl | 2-Trifluormethylphenyl | CH | |
| 100 | Phenyl | 3-Trifluormethylphenyl | N | |
| 101 | Phenyl | 3-Trifluormethylphenyl | CH | |
| 102 | Phenyl | 4-Trifluormethylphenyl | N | |
| 103 | Phenyl | 4-Trifluormethylphenyl | CH | |
| 104 | Phenyl | 1-Naphthyl | N | |
| 105 | Phenyl | 2-Naphthyl | N | |
| 106 | Phenyl | Cyclohexyl | N | |
| 107 | Phenyl | Cyclohexyl | CH | |
| 108 | 2-Chlorphenyl | Phenyl | N | |
| 109 | 2-Chlorphenyl | Phenyl | CH | |
| 110 | 2-Chlorphenyl | 2-Chlorphenyl | N | |
| 111 | 2-Chlorphenyl | 2-Chlorphenyl | CH | |
| 112 | 2-Chlorphenyl | 4-Chlorphenyl | N | |
| 113 | 2-Chlorphenyl | 4-Chlorphenyl | CH | |
| 114 | 2-Chlorphenyl | 2,4-Dichlorphenyl | N | |
| 115 | 2-Chlorphenyl | 2,4-Dichlorphenyl | CH | |
| 116 | 2-Chlorphenyl | 2-Fluorphenyl | N | |

| Bsp | A | B | X | physikalische Daten Schmp./IR |
|---|---|---|---|---|
| 117 | 2-Chlorphenyl | 2-Fluorphenyl | CH | |
| 118 | 2-Chlorphenyl | 3-Fluorphenyl | N | |
| 119 | 2-Chlorphenyl | 3-Fluorphenyl | CH | |
| 120 | 2-Chlorphenyl | 4-Fluorphenyl | N | |
| 121 | 2-Chlorphenyl | 4-Fluorphenyl | CH | |
| 122 | 2-Chlorphenyl | 2-Trifluormethylphenyl | N | |
| 123 | 2-Chlorphenyl | 2-Trifluormethylphenyl | CH | |
| 124 | 2-Chlorphenyl | 3-Trifluormethylphenyl | N | |
| 125 | 2-Chlorphenyl | 3-Trifluormethylphenyl | CH | |
| 126 | 2-Chlorphenyl | 4-Trifluormethylphenyl | N | |
| 127 | 2-Chlorphenyl | 4-Trifluormethylphenyl | CH | |
| 128 | 2-Chlorphenyl | 2-Methylphenyl | N | |
| 129 | 2-Chlorphenyl | 2-Methylphenyl | CH | |
| 130 | 2-Chlorphenyl | 4-Methylphenyl | N | |
| 131 | 2-Chlorphenyl | 4-Methylphenyl | CH | |
| 132 | 2-Chlorphenyl | 4-Biphenyl | N | |
| 133 | 2-Chlorphenyl | Cyclohexyl | N | |
| 134 | 4-Chlorphenyl | Phenyl | N | |
| 135 | 4-Chlorphenyl | Phenyl | CH | |
| 136 | 4-Chlorphenyl | 2-Chlorphenyl | N | 214-215°C |
| 137 | 4-Chlorphenyl | 2-Chlorphenyl | CH | |
| 138 | 4-Chlorphenyl | 3-Chlorphenyl | N | |
| 139 | 4-Chlorphenyl | 3-Chlorphenyl | CH | |
| 140 | 4-Chlorphenyl | 4-Chlorphenyl | N | |
| 141 | 4-Chlorphenyl | 4-Chlorphenyl | CH | |
| 142 | 4-Chlorphenyl | 2,4-Dichlorphenyl | N | |
| 143 | 4-Chlorphenyl | 2,4-Dichlorphenyl | CH | |
| 144 | 4-Chlorphenyl | 2-Fluorphenyl | N | 116°C |
| 145 | 4-Chlorphenyl | 2-Fluorphenyl | CH | |
| 146 | 4-Chlorphenyl | 3-Fluorphenyl | N | |
| 147 | 4-Chlorphenyl | 3-Fluorphenyl | CH | |
| 148 | 4-Chlorphenyl | 4-Fluorphenyl | N | |
| 149 | 4-Chlorphenyl | 4-Fluorphenyl | CH | |
| 150 | 4-Chlorphenyl | 2-Bromphenyl | N | |
| 151 | 4-Chlorphenyl | 2-Bromphenyl | CH | |
| 152 | 4-Chlorphenyl | 3-Bromphenyl | N | |
| 153 | 4-Chlorphenyl | 3-Bromphenyl | CH | |
| 154 | 4-Chlorphenyl | 4-Bromphenyl | N | |
| 155 | 4-Chlorphenyl | 4-Bromphenyl | CH | |
| 156 | 4-Chlorphenyl | 2-Trifluormethylphenyl | N | |
| 157 | 4-Chlorphenyl | 2-Trifluormethylphenyl | CH | |

| Bsp | A | B | X | physikalische Daten Schmp./IR |
|---|---|---|---|---|
| 158 | 4-Chlorphenyl | 3-Trifluormethylphenyl | N | |
| 159 | 4-Chlorphenyl | 3-Trifluormethylphenyl | CH | |
| 160 | 4-Chlorphenyl | 4-Trifluormethylphenyl | N | |
| 161 | 4-Chlorphenyl | 4-Trifluormethylphenyl | CH | |
| 162 | 4-Chlorphenyl | 2-Methylphenyl | N | |
| 163 | 4-Chlorphenyl | 2-Methylphenyl | CH | |
| 164 | 4-Chlorphenyl | 4-Methylphenyl | N | |
| 165 | 4-Chlorphenyl | 4-Methylphenyl | CH | |
| 166 | 4-Chlorphenyl | 1-Naphthyl | N | |
| 167 | 4-Chlorphenyl | 2-Naphthyl | N | |
| 168 | 4-Chlorphenyl | 4-Tetrahydropyranyl | N | |
| 169 | 4-Chlorphenyl | Cyclopropyl | N | |
| 170 | 4-Chlorphenyl | Cyclopropyl | CH | |
| 171 | 4-Chlorphenyl | Cyclopentyl | N | |
| 172 | 4-Chlorphenyl | Cyclopentyl | CH | |
| 173 | 4-Chlorphenyl | Cyclohexyl | N | |
| 174 | 4-Chlorphenyl | Cyclohexyl | CH | |
| 175 | 2,4-Dichlorphenyl | Phenyl | N | |
| 176 | 2,4-Dichlorphenyl | Phenyl | CH | |
| 177 | 2,4-Dichlorphenyl | 2-Chlorphenyl | N | |
| 178 | 2,4-Dichlorphenyl | 2-Chlorphenyl | CH | |
| 179 | 2,4-Dichlorphenyl | 3-Chlorphenyl | N | |
| 180 | 2,4-Dichlorphenyl | 3-Chlorphenyl | CH | |
| 181 | 2,4-Dichlorphenyl | 4-Chlorphenyl | N | |
| 182 | 2,4-Dichlorphenyl | 4-Chlorphenyl | CH | |
| 183 | 2,4-Dichlorphenyl | 2,4-Dichlorphenyl | N | |
| 184 | 2,4-Dichlorphenyl | 2,4-Dichlorphenyl | CH | |
| 185 | 2,4-Dichlorphenyl | 2-Fluorphenyl | N | |
| 186 | 2,4-Dichlorphenyl | 2-Fluorphenyl | CH | |
| 187 | 2,4-Dichlorphenyl | 3-Fluorphenyl | N | |
| 188 | 2,4-Dichlorphenyl | 3-Fluorphenyl | CH | |
| 189 | 2,4-Dichlorphenyl | 4-Fluorphenyl | N | |
| 190 | 2,4-Dichlorphenyl | 4-Fluorphenyl | CH | |
| 191 | 2,4-Dichlorphenyl | 2-Bromphenyl | N | |
| 192 | 2,4-Dichlorphenyl | 2-Bromphenyl | CH | |
| 193 | 2,4-Dichlorphenyl | 2-Trifluormethylphenyl | N | |
| 194 | 2,4-Dichlorphenyl | 2-Trifluormethylphenyl | CH | |
| 195 | 2,4-Dichlorphenyl | 3-Trifluormethylphenyl | N | |
| 196 | 2,4-Dichlorphenyl | 3-Trifluormethylphenyl | CH | |
| 197 | 2,4-Dichlorphenyl | 4-Trifluormethylphenyl | N | |
| 198 | 2,4-Dichlorphenyl | 4-Trifluormethylphenyl | CH | |

| Bsp | A | B | X | physikalische Daten Schmp./IR |
|---|---|---|---|---|
| 199 | 2,4-Dichlorphenyl | 2-Methylphenyl | N | |
| 200 | 2,4-Dichlorphenyl | 2-Methylphenyl | CH | |
| 201 | 2,4-Dichlorphenyl | Cyclohexyl | N | |
| 202 | 2,4-Dichlorphenyl | Cyclohexyl | CH | |
| 203 | 2-Fluorphenyl | Phenyl | N | |
| 204 | 2-Fluorphenyl | 2-Chlorphenyl | N | |
| 205 | 2-Fluorphenyl | 4-Chlorphenyl | N | |
| 206 | 2-Fluorphenyl | 2,4-Dichlorphenyl | N | |
| 207 | 2-Fluorphenyl | 4-Fluorphenyl | N | |
| 208 | 2-Fluorphenyl | 4-Bromphenyl | N | |
| 209 | 2-Fluorphenyl | 2-Trifluormethylphenyl | N | |
| 210 | 2-Fluorphenyl | 4-Trifluormethylphenyl | N | |
| 211 | 2-Fluorphenyl | 2-Methylphenyl | N | |
| 212 | 2-Fluorphenyl | Cyclopentyl | N | |
| 213 | 4-Bromphenyl | Phenyl | N | |
| 214 | 4-Bromphenyl | Phenyl | CH | |
| 215 | 4-Bromphenyl | 2-Chlorphenyl | N | |
| 216 | 4-Bromphenyl | 3-Chlorphenyl | N | |
| 217 | 4-Bromphenyl | 4-Chlorphenyl | N | |
| 218 | 4-Bromphenyl | 2,4-Dichlorphenyl | N | |
| 219 | 4-Bromphenyl | 2-Trifluormethylphenyl | N | |
| 220 | 4-Bromphenyl | 4-Trifluormethylphenyl | N | |
| 221 | 4-Bromphenyl | 2-Methylphenyl | N | |
| 222 | 2-Trifluor-methylphenyl | Phenyl | N | |
| 223 | 2-Trifluor-methylphenyl | 2-Chlorphenyl | N | |
| 224 | 2-Trifluor-methylphenyl | 4-Chlorphenyl | N | |
| 225 | 2-Trifluor-methylphenyl | 2,4-Dichlorphenyl | N | |
| 226 | 2-Trifluor-methylphenyl | 2-Trifluormethylphenyl | N | |
| 227 | 2-Trifluor-methylphenyl | 4-Trifluormethylphenyl | N | |
| 228 | 2-Trifluor-methylphenyl | 2-Fluorphenyl | N | |
| 229 | 2-Trifluor-methylphenyl | 4-Fluorphenyl | N | |
| 230 | 2-Trifluor-methylphenyl | 2-Methylphenyl | N | |

| Bsp | A | B | X | physikalische Daten Schmp./IR |
|---|---|---|---|---|
| 231 | 4-Trifluor-methylphenyl | Phenyl | N | |
| 232 | 4-Trifluor-methylphenyl | Phenyl | CH | |
| 233 | 4-Trifluor-methylphenyl | 2-Chlorphenyl | N | |
| 234 | 4-Trifluor-methylphenyl | 4-Chlorphenyl | N | |
| 235 | 4-Trifluor-methylphenyl | 2-Fluorphenyl | N | |
| 236 | 4-Trifluor-methylphenyl | 4-Fluorphenyl | N | |
| 237 | 4-Trifluor-methylphenyl | 2,4-Dichlorphenyl | N | |
| 238 | 4-Trifluor-methylphenyl | 2-Bromphenyl | N | |
| 239 | 4-Trifluor-methylphenyl | 2-Trifluormethylphenyl | N | |
| 240 | 4-Trifluor-methylphenyl | 4-Trifluormethylphenyl | N | |
| 241 | 4-Trifluor-methylphenyl | 2-Methylphenyl | N | |
| 242 | 4-Trifluor-methylphenyl | 4-Methylphenyl | N | |
| 243 | 2-Methylphenyl | Phenyl | N | |
| 244 | 2-Methylphenyl | 2-Chlorphenyl | N | |
| 245 | 2-Methylphenyl | 4-Chlorphenyl | N | |
| 246 | 2-Methylphenyl | 2-Fluorphenyl | N | |
| 247 | 2-Methylphenyl | 4-Fluorphenyl | N | |
| 248 | 2-Methylphenyl | 4-Trifluormethylphenyl | N | |
| 249 | 4-Methylphenyl | Phenyl | N | |
| 250 | 4-Methylphenyl | 2-Chlorphenyl | N | |
| 251 | 4-Methylphenyl | 4-Chlorphenyl | N | |
| 252 | 4-Methylphenyl | 2-Fluorphenyl | N | |
| 253 | 4-Methylphenyl | 4-Fluorphenyl | N | |
| 254 | 4-Methylphenyl | 4-Trifluormethylphenyl | N | |
| 255 | 4-tert.-Butyl-phenyl | Phenyl | N | |
| 256 | 4-tert.-Butyl-phenyl | 2-Chlorphenyl | N | |

| Bsp | A | B | X | physikalische Daten Schmp./IR |
|---|---|---|---|---|
| 257 | 4-tert.-Butyl-phenyl | 2-Fluorphenyl | N | |
| 258 | 2-Methoxyphenyl | Phenyl | N | |
| 259 | 2-Methoxyphenyl | 2-Chlorphenyl | N | |
| 260 | 2-Methoxyphenyl | 4-Chlorphenyl | N | |
| 261 | 2-Methoxyphenyl | 2-Fluorphenyl | N | |
| 262 | 2-Methoxyphenyl | 4-Fluorphenyl | N | |
| 263 | 4-Methoxyphenyl | Phenyl | N | |
| 264 | 4-Methoxyphenyl | 2-Chlorphenyl | N | |
| 265 | 4-Methoxyphenyl | 4-Chlorphenyl | N | |
| 266 | 4-Methoxyphenyl | 2-Fluorphenyl | N | |
| 267 | 4-Methoxyphenyl | 4-Fluorphenyl | N | |
| 268 | 4-Methoxyphenyl | 2,4-Dichlorphenyl | N | |
| 269 | 4-Biphenyl | Phenyl | N | |
| 270 | 4-Biphenyl | 2-Chlorphenyl | N | |
| 271 | 4-Biphenyl | 4-Chlorphenyl | N | |
| 272 | 4-Biphenyl | 2-Fluorphenyl | N | |
| 273 | 4-Biphenyl | 4-Fluorphenyl | N | |
| 274 | 4-Phenoxyphenyl | Phenyl | N | |
| 275 | 4-Phenoxyphenyl | 2-Chlorphenyl | N | |
| 276 | 4-Phenoxyphenyl | 4-Chlorphenyl | N | |
| 277 | 4-Phenoxyphenyl | 4-Fluorphenyl | N | |
| 278 | 1-Naphthyl | 2-Chlorphenyl | N | |
| 279 | 1-Naphthyl | 4-Chlorphenyl | N | |
| 280 | 1-Naphthyl | 4-Fluorphenyl | N | |
| 281 | 2-Naphthyl | 4-Phenoxyphenyl | N | |
| 282 | 2-Naphthyl | 2-Chlorphenyl | N | |
| 283 | 2-Naphthyl | 4-Chlorphenyl | N | |
| 284 | 2-Naphthyl | 4-Fluorphenyl | N | |
| 285 | 4-Tetrahydro-pyranyl | 2-Chlorphenyl | N | |
| 286 | 4-Tetrahydro-pyranyl | 4-Chlorphenyl | N | |
| 287 | 4-Tetrahydro-pyranyl | 2-Fluorphenyl | N | |
| 288 | 4-Tetrahydro-pyranyl | 4-Fluorphenyl | N | |
| 289 | 4-Tetrahydro-pyranyl | 2,4-Dichlorphenyl | N | |
| 290 | Methyl | Phenyl | N | |
| 291 | Methyl | 2-Chlorphenyl | N | |

| Bsp | A | B | X | physikalische Daten Schmp./IR |
|---|---|---|---|---|
| 292 | Methyl | 4-Chlorphenyl | N | |
| 293 | Methyl | 2,4-Dichlorphenyl | N | |
| 294 | Methyl | 2-Fluorphenyl | N | |
| 295 | Methyl | 4-Fluorphenyl | N | |
| 296 | Methyl | 2-Bromphenyl | N | |
| 297 | Methyl | 4-Trifluormethylphenyl | N | |
| 298 | Methyl | 2-Trifluormethylphenyl | N | |
| 299 | tert.-Butyl | Phenyl | N | |
| 300 | tert.-Butyl | Phenyl | CH | |
| 301 | tert.-Butyl | 2-Chlorphenyl | N | |
| 302 | tert.-Butyl | 2-Chlorphenyl | CH | |
| 303 | tert.-Butyl | 4-Chlorphenyl | N | |
| 304 | tert.-Butyl | 4-Chlorphenyl | CH | |
| 305 | tert.-Butyl | 2,4-Dichlorphenyl | N | |
| 306 | tert.-Butyl | 2,4-Dichlorphenyl | CH | |
| 307 | tert.-Butyl | 4-Fluorphenyl | N | |
| 308 | tert.-Butyl | 4-Fluorphenyl | CH | |
| 309 | tert.-Butyl | 2-Fluorphenyl | N | |
| 310 | tert.-Butyl | 2-Fluorphenyl | CH | |
| 311 | tert.-Butyl | 4-Trifluormethylphenyl | N | |
| 312 | tert.-Butyl | 4-Trifluormethylphenyl | CH | |
| 313 | tert.-Butyl | 2-Bromphenyl | N | |
| 314 | tert.-Butyl | 2-Bromphenyl | CH | |
| 315 | tert.-Butyl | 2-Methylphenyl | N | |
| 316 | tert.-Butyl | 2-Methylphenyl | CH | |
| 317 | tert.-Butyl | 1-Naphthyl | N | |
| 318 | tert.-Butyl | 2-Naphthyl | N | |
| 319 | tert.-Butyl | Cyclohexyl | N | |
| 320 | tert.-Butyl | Cyclohexyl | CH | |
| 321 | Cyclohexyl | Phenyl | N | |
| 322 | Cyclohexyl | Phenyl | CH | |
| 323 | Cyclohexyl | 2-Chlorphenyl | N | |
| 324 | Cyclohexyl | 2-Chlorphenyl | CH | |
| 325 | Cyclohexyl | 4-Chlorphenyl | N | |
| 326 | Cyclohexyl | 4-Chlorphenyl | CH | |
| 327 | Cyclohexyl | 2,4-Dichlorphenyl | N | |
| 328 | Cyclohexyl | 2,4-Dichlorphenyl | CH | |
| 329 | Cyclohexyl | 2-Fluorphenyl | N | |
| 330 | Cyclohexyl | 2-Fluorphenyl | CH | |
| 331 | Cyclohexyl | 4-Fluorphenyl | N | |
| 332 | Cyclohexyl | 4-Fluorphenyl | CH | |

| Bsp | A | B | X | physikalische Daten Schmp./IR |
|---|---|---|---|---|
| 333 | Cyclohexyl | 2-Trifluormethylphenyl | N | |
| 334 | Cyclohexyl | 2-Trifluormethylphenyl | CH | |
| 335 | Cyclohexyl | 4-Trifluormethylphenyl | N | |
| 336 | Cyclohexyl | 4-Trifluormethylphenyl | CH | |
| 337 | Cyclohexyl | 2-Bromphenyl | N | |
| 338 | Cyclohexyl | 2-Bromphenyl | CH | |
| 339 | Cyclohexyl | 2-Methylphenyl | N | |
| 340 | Cyclohexyl | 2-Methylphenyl | CH | |
| 341 | Cyclohexyl | 4-Biphenyl | N | |
| 342 | Cyclohexyl | 2-Naphthyl | N | |
| 343 | Cyclohexyl | 2-Methoxyphenyl | N | |
| 344 | Cyclohexyl | Cyclohexyl | N | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora Infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe

kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1 werden in einer Mischung gelöst, die aus 25 Gew.-Teilen (Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die N-Oxo-azolylmethyloxirane der allgemeinen Formel I können praktisch alle Entwicklungsstadien einer Pflanze verschiedenartig beeinflussen und werden deshalb als Bioregulatoren eingesetzt. Die Wirkungsvielfalt der Bioregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und

g) von den angewendeten Konzentrationen der aktiven Substanz.

Aus der Reihe der verschiedenartigen Anwendungsmöglichkeiten der erfindungsgemäßen Bioregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanzen ermöglicht.

Durch Anwendung der Verbindungen I kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den Verbindungen I läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den Mitteln auf der Basis von N-Oxo-azolylmethyloxiranen I lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die N-Oxo-azolylmethyloxirane der Formel I können Ertragssteigerungen durch Eingriffe in den pflanzlichen Stoffwechsel bzw. durch Förderung oder Hemmung des vegetativen und/oder des generativen Wachstums verursachen.

C. Mit den N-Oxoa-zolylmethyloxiranen I lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt- und Sproßachse ist auch für ein gut kontrollierbares Entblättern von Nutzpflanzen z.B. Baumwolle wesentlich.

D. Mit den N-Oxo-azolylmethyloxiranen I kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden

- die Durchwurzelung des Bodens verbessert wird
- das Mikroklima im Pflanzenbestand durch einen kompakteren Wuchs günstig beeinflußt wird.

Die Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Verträglichkeit der Pflanzen für die Verbindungen I kann die Aufwandmenge stark variiert werden. Bei der Saatgutbehandlung werden im allgemeinen Wirkstoffmengen von 0,001 bis 50 g je Kilogramm Saatgut, vorzugsweise 0,01 bis 1 g, benötigt. Für die Blatt- und Bodenbehandlung sind im allgemeinen Gaben von 0,01 bis 10 kg/ha, bevorzugt 0,1 bis 5 kg/ha ausreichend.

Die Formulierungen enthalten im allgemeinen zwischen 0,1 und 95 Gew.% Wirkstoff, vorzugsweise zwischen 0,5 und 90 Gew.%.

Die Mittel auf der Basis von Azolylmethyloxiranen I können in diesen Anwendungsformen auch zusammen mit anderen Wirkstoffen vorliegen, wie z.B. Herbiziden, Insektiziden, anderen Wachstumsregulatoren und Fungiziden, oder auch mit Düngemitteln vermischt und ausgebracht werden, wobei auch synergistische Effekte auftreten können, d.h. die Wirksamkeit des Kombinationsproduktes ist größer als die addierten Wirksamkeiten der Einzelkomponenten.

## Ansprüche

1. N-Oxo-Azolylmethyloxirane der allgemeinen Formel I

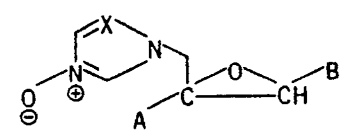

I,

in welcher
A und B gleich oder verschieden $C_1$-$C_8$-Alkyl, $C_3$-$C_8$-(Cycloalkyl, Tetrahydropyranyl, Benzyl, Norbornyl, Naphthyl, Biphenyl oder Phenyl bedeuten, wobei diese Reste unsubstituiert oder ein- bis dreifach durch Halogen, Nitro, Phenoxy, $C_1$-$C_4$-Alkyl, $C_1$-$C_4$-Alkoxy oder $C_1$-$C_4$-Halogenalkyl substituiert sind und
X für CH oder N steht.

2. N-Oxo-azolylmethyloxirane der allgemeinen Formel I gemäß Anspruch 1, in der A und B Phenyl oder einen durch Fluor oder Chlor substituierten Phenylrest bedeuten.

3. Verfahren zur Herstellung eines N-Oxo-azolylmethyloxirans der Formel I

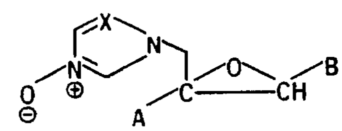

I,

in welcher
A und B gleich oder verschieden $C_1$-$C_8$-Alkyl, $C_3$-$C_8$-Cycloalkyl, Tetrahydropyranyl, Benzyl, Norbornyl, Naphthyl, Biphenyl oder Phenyl bedeuten, wobei diese Reste unsubstituiert oder ein- bis dreifach durch Halogen, Nitro, Phenoxy, $C_1$-$C_4$-Alkyl, $C_1$-$C_4$-Alkoxy oder $C_1$-$C_4$-Halogenalkyl substituiert sind und
X für CH oder N steht,
dadurch gekennzeichnet, daß man Azolylmethyloxirane der Formel II

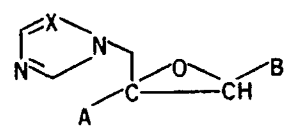

II,

in welcher A, B und X die in Anspruch 1 angegebene Bedeutung haben, mit Peroxycarbonsäuren, $H_2O_2$ oder Alkylhydroperoxyden in Gegenwart eines indifferenten Lösungsmittels zu den N-Oxyden I umsetzt.

4. Fungizides Mittel, enthaltend einen Trägerstoff und ein N-Oxo-Azolylmethyloxiran der Formel I

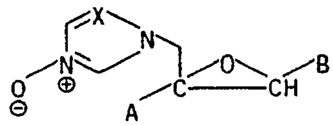

I,

in welcher
A und B gleich oder verschieden $C_1$-$C_8$-Alkyl, $C_3$-$C_8$-Cycloalkyl, Tetrahydropyranyl, Norbornyl, Naphthyl, Biphenyl oder Phenyl bedeuten, wobei diese Reste unsubstituiert oder ein- bis dreifach durch Halogen, Nitro, Phenoxy, $C_1$-$C_4$-Alkyl, $C_1$-$C_4$-Alkoxy oder $C_1$-$C_4$-Halogenalkyl substituiert sind und
X für CH oder N steht.

5. Verfahren zur Bekämpfung von Pilzen, dadurch gekennzeichnet, daß man eine fungizid wirksame Menge eines N-Oxo-Azolylmethyloxirans der allgemeinen Formel I

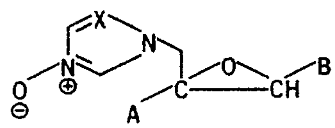

I,

in welcher
A und B gleich oder verschieden $C_1$-$C_8$-Alkyl, $C_3$-$C_8$-Cycloalkyl, Tetrahydropyranyl, Norbornyl, Naphthyl, Biphenyl oder Phenyl bedeuten, wobei diese Reste unsubstituiert oder ein- bis dreifach durch Halogen, Nitro, Phenoxy, $C_1$-$C_4$-Alkyl, $C_1$-$C_4$-Alkoxy oder $C_1$-$C_4$-Halogenalkyl substituiert sind und
X für CH oder N steht, auf die Pilze oder durch Pilzbefall bedrohte Materialien, Flächen, Pflanzen oder Saatgüter einwirken läßt.

6. Mittel zur Regulierung des Pflanzenwachstums, enthaltend einen Trägerstoff und ein N-Oxo-Azolylmethyloxiran der Formel I

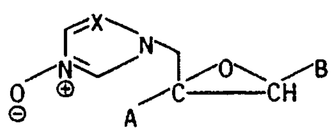

I,

in welcher
A und B gleich oder verschieden $C_1$-$C_8$-Alkyl, $C_3$-$C_8$-Cycloalkyl, Tetrahydropyranyl, Norbornyl, Naphthyl, Biphenyl oder Phenyl bedeuten, wobei diese Reste unsubstituiert oder ein- bis dreifach durch Halogen, Nitro, Phenoxy, $C_1$-$C_4$-Alkyl, $C_1$-$C_4$-Alkoxy oder $C_1$-$C_4$-Halogenalkyl substituiert sind und
X für CH oder N steht.

7. Verfahren zur Regulierung des Pflanzenwachstums, dadurch gekennzeichnet, daß man den Boden, das Saatgut und/oder die Pflanzen mit einem N-Oxo-Azolylmethyloxiran der Formel I gemäß Anspruch 1 behandelt.